# EUROPEAN PATENT SPECIFICATION

(11) **EP 4 216 841 B1**
(45) Date of publication and mention of the grant of the patent: **12.02.2025**
(21) Application number: 21786254.9
(22) Date of filing: 07.09.2021
(51) Int. Cl.: A61B 17/29, A61B 10/06, A61B 17/16, A61B 17/295, A61B 17/32, A61B 17/34

(54) **CUTTING INSTRUMENT WITH SAFETY SLEEVE**
SCHNEIDINSTRUMENT MIT SICHERHEITSHÜLSE
INSTRUMENT DE COUPE POURVU D'UN MANCHON DE SÉCURITÉ

(30) Priority: 24.09.2020 GB 202015085
(43) Date of publication of application: 02.08.2023
(73) Proprietor: Jessup, Mark, Stocksfield, Northumberland NE43 7QX (GB)
(72) Inventor: Jessup, Mark, Stocksfield, Northumberland NE43 7QX (GB)
(74) Representative: Hartley, Lucinda Jane
(86) International application number: PCT/GB2021/052295
(87) International publication number: WO 2022/064169

(56) References cited:
- EP-A2- 1 051 946
- WO-A1-2015/164591
- US-A1- 2008 039 883

## Description

### Field of the Invention

The present invention relates to surgical instruments including a sharp cutting tip which include a safety sleeve for protecting the cutting tip of the instrument after use.

### Background of the Invention

Cutting instruments used in certain surgical procedures, such as biopsy punches or laparascopic scissors, have a sharp cutting tip to allow for the cutting of tissues and/or removal of tissue samples from patients. Needlestick and other sharps injuries are a serious hazard to health professionals, particularly once the equipment has been contaminated with potentially hazardous biological material.

It would be desirable to provide a cutting instrument which includes an integrated safety sleeve to shield the cutting tip after use. US2008/039883 A1, WO 2015/164591 A1 and EP1051946 A2 discloses devices of the prior art.

### Summary of the Invention

Claim 1 defines the invention and dependent claims disclose embodiments. No surgical methods are claimed. According to a first aspect of the disclosure there is provided a surgical cutting instrument comprising a handle portion and an elongate shaft, the shaft having a distal end and a proximal end and a longitudinal axis extending therebetween, the shaft mounting a cutting element at the distal end, wherein the shaft is rotatably mounted onto the handle portion at the proximal end, allowing for rotation of the shaft about the longitudinal axis, the instrument further comprising a safety sleeve including a substantially cylindrical channel through which the elongate shaft extends, wherein the safety sleeve is moveable axially along the shaft between the proximal end and the distal end of the shaft, and wherein the instrument further comprises locking means for preventing further axial movement of the safety sleeve with respect to the elongate shaft when the safety sleeve is in the extended position; The locking means comprises first and second cooperating elements each located on a respective one of the distal end of the shaft and the safety sleeve.

The surgical cutting instrument may be selected from the group comprising: biopsy apparatus and laparoscopic scissors.

The locking means automatically engages when the safety sleeve is moved over the cutting tip. Once the locking means has engaged the safety sleeve cannot then be retracted to expose the cutting tip. The locking means is engaged when the first and second cooperating elements interact.

Preferably, when the safety sleeve is in the retracted position, rotation of the safety sleeve is configured to rotate the elongate shaft.

Preferably the instrument further comprises means for maintaining a static connection between the safety sleeve and the elongate shaft when the safety sleeve is in the retracted position, and preferably the means for maintaining a static connection comprises third and fourth cooperating elements each located on a respective one of the proximal end of the shaft and the safety sleeve.

Preferably, the third and fourth cooperating elements comprise male/female cooperating elements. More preferably, one of the third and fourth cooperating elements is a protrusion and the other of the third and fourth cooperating elements is a slot shaped to accommodate the protrusion.

The first cooperating element may comprise a collar located at the distal end of the elongate shaft, adjacent the cutting tip and the second cooperating element may comprise at least one resilient tab located on the safety sleeve and an annular channel shaped to receive the collar located within the cylindrical channel of the safety sleeve.

Preferably the safety sleeve includes a plurality of resilient tabs.

Preferably, the at least one resilient tab includes a protrusion and one edge of the annular channel is defined by the protrusion.

Alternatively, the first cooperating element may comprise at least one resilient clip including a protrusion located on the safety sleeve and biased towards the elongate shaft; and the second cooperating element may comprise at least one aperture for receiving the or each protrusion located at the distal end of the elongate shaft, adjacent the cutting tip, such that in the extended position the or each protrusion locates within the or each aperture preventing further axial movement of the safety sleeve with respect to the elongate shaft.

Preferably, the safety sleeve includes a plurality of resilient clips, each including a protrusion.

Preferably, the safety sleeve further comprises a portion of increased diameter located adjacent the proximal end of the elongate shaft. Preferably, the portion of the safety sleeve with increased diameter is configured to be gripped and includes a plurality of ridges. Preferably, the surgical cutting instrument is for extracting a biopsy sample. According to a second aspect of the disclosure, there is provided an exemplary method of using a surgical cutting instrument as hereinbefore defined, with the safety sleeve located in the retracted position, the method comprising the steps of:
a) ensuring the safety sleeve of the surgical instrument is located in the retracted position;
b) operating the cutting tip; and
c) moving the safety sleeve along the elongate shaft towards the cutting tip until the cutting tip is covered by the safety sleeve and the locking means is engaged.

The elongate shaft may be rotated to orient the cutting tip in the desired direction before operating the cutting tip.

Where the instrument is for extracting a biopsy sample the method includes the step of removing a tissue sample after step b).

The invention provides an improved surgical cutting instrument which includes an integrated locking safety sleeve to protect users from accidental sharps injuries. By integrating the safety sleeve into the apparatus, the risk of injury through accidental contact with the used cutting tip is minimized.

### Brief Description of the drawings

In the Drawings, which illustrate preferred embodiments of cutting apparatus, are by way of example:
Figure 1 illustrates a biopsy apparatus according to a first embodiment of the invention, with a safety sleeve in a retracted position;
Figure 2 illustrates the biopsy apparatus of Figure 1 with the safety sleeve in an extended position;
Figure 3 is an exploded view of the proximal end of the apparatus of Figure 1, illustrating the connection between the safety sleeve and the elongate shaft of the apparatus;
Figure 4 illustrates a longitudinal cross-sectional view through the safety sleeve of the apparatus of Figure 1, with the safety sleeve in a retracted position;
Figure 5 illustrates a longitudinal cross-sectional view through the cutting tip of the apparatus of Figure 1;
Figure 6 illustrates a cross-sectional view through the safety sleeve of the apparatus of Figure 2, with the safety sleeve in the extended position;
Figure 7 illustrates a cross-sectional view through the safety sleeve of an alternative embodiment of the apparatus of the invention Figure 2, with the safety sleeve in the retracted position;
Figure 8 is a close up view of the cutting tip at the distal end of the apparatus of Figure 7; and
Figure 9 illustrates a cross-sectional view through the safety sleeve of the apparatus of Figure 7, with the safety sleeve in the extended position.

### Detailed Description of the Preferred Embodiments

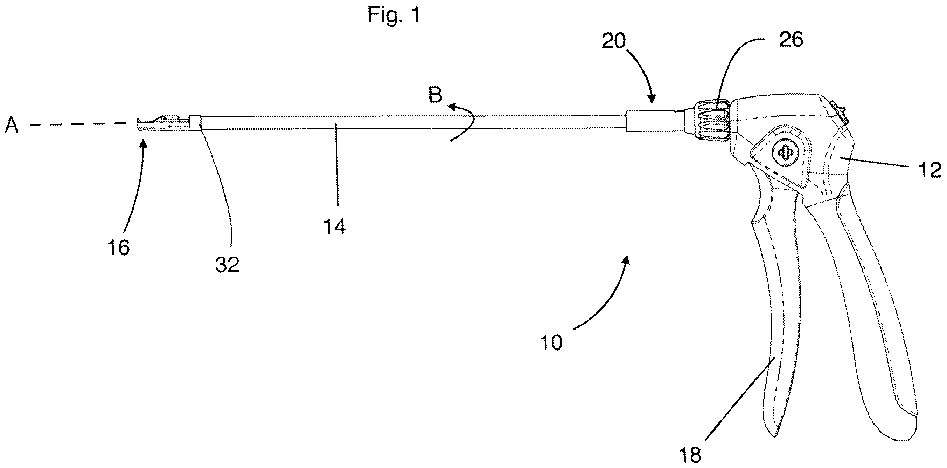

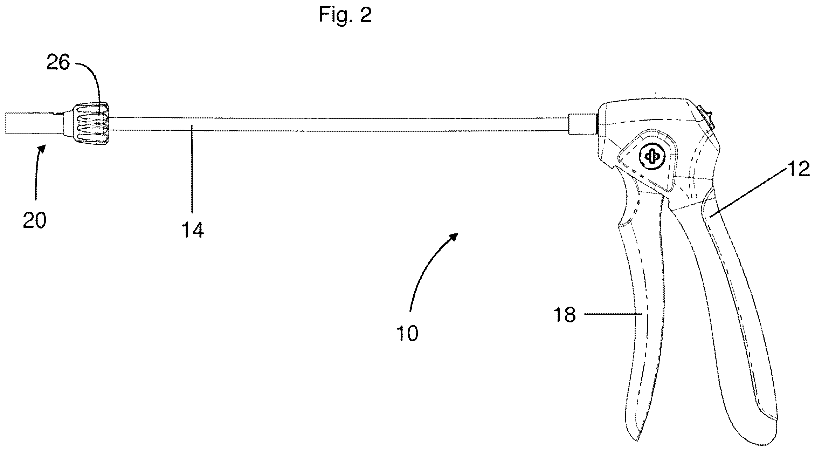

Referring now to Figures 1 and 2, a biopsy apparatus is shown generally at 10 comprises a handle portion 12 and an elongate shaft 14 connected to the handle 12 at the proximal end of the shaft 14. A cutting tip 16 is mounted at the distal end of the shaft 14. The distal end of the shaft 14 is the end of the shaft 14 that is furthest away from the handle. The proximal end of the shaft 14 is the end of the shaft 14 that is closest to the handle. The elongate shaft 14 is rotatably mounted on the handle portion 12 so that it may be rotated about its longitudinal axis (labelled 'A' in Figure 1) as shown generally by the arrow marked B in Figure 1. Rotation of the elongate shaft 14 allows the health professional to correctly orient the cutting tip 16 of the apparatus 10 during the biopsy procedure. The cutting tip 16 is operated using the trigger 18 on the handle portion 12. The mechanism for operation of the cutting tip 16 does not form part of the current invention and would be well known to the skilled person.

The apparatus further comprises a safety sleeve 20 which includes a substantially cylindrical channel through which the elongate shaft 14 is mounted. The safety sleeve 20 can be moved axially along the longitudinal axis of the shaft 14 between the proximal and distal ends of the shaft 14. Figure 1 illustrates the safety sleeve 20 in a retracted position, located at the proximal end of the shaft 14. Figure 2 illustrates the safety sleeve 20 in an extended position, covering the cutting tip 16 at the distal end of the shaft 14. The safety sleeve 20 is moved between the retracted and extended position manually.

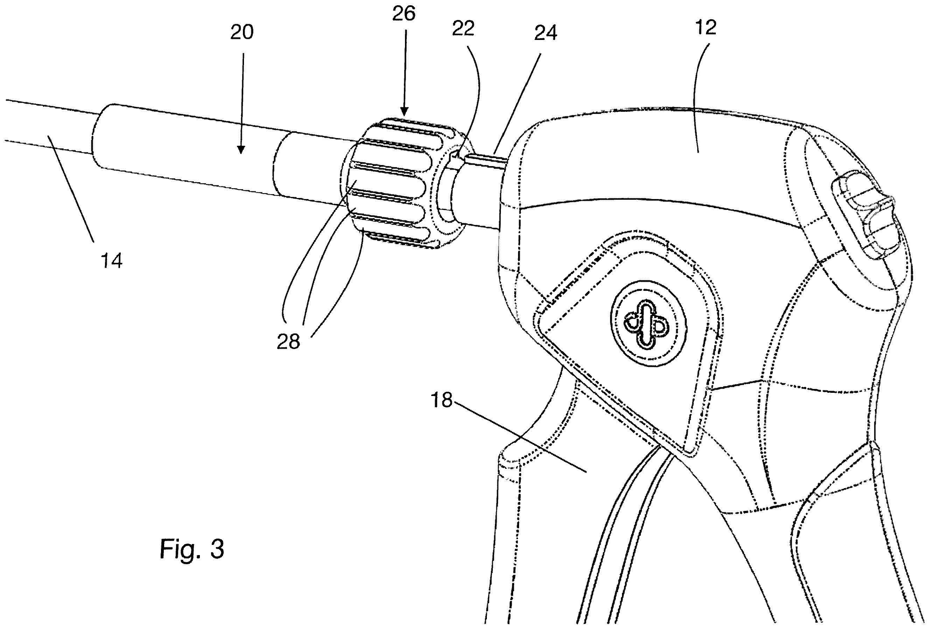

As shown in Figure 3, the safety sleeve 20 and base of the shaft 14 include cooperating means, in the illustrated example the cooperating means is in the form of a slot 22 on the safety sleeve and a correspondingly shaped protrusion 24 at the proximal end of the shaft 14. The cooperating means ensures a static connection between the shaft 14 and the safety sleeve when the safety sleeve 20 is in the retracted position, and this allows the shaft 14 to be rotated by a user manually gripping and rotating the safety sleeve 20. An alternative combination of male/female connectors could be used to achieve the same result. Preferably, as shown in the figures, the safety sleeve 20 include a base portion 26 of increased diameter, and preferably includes a ribbed surface 28 for enhanced grip. The increased diameter of the base portion 26 provides an easily grippable portion of the sleeve 20 that can be used to rotate the shaft 14, and also serves as a shield to protect the fingers of the user when the safety sleeve is moved along the shaft towards the cutting tip.

The apparatus 10 further includes a locking mechanism to prevent retraction of the safety sleeve 20 once it has been moved to the extended position, covering the cutting tip, as shown in Figure 2.

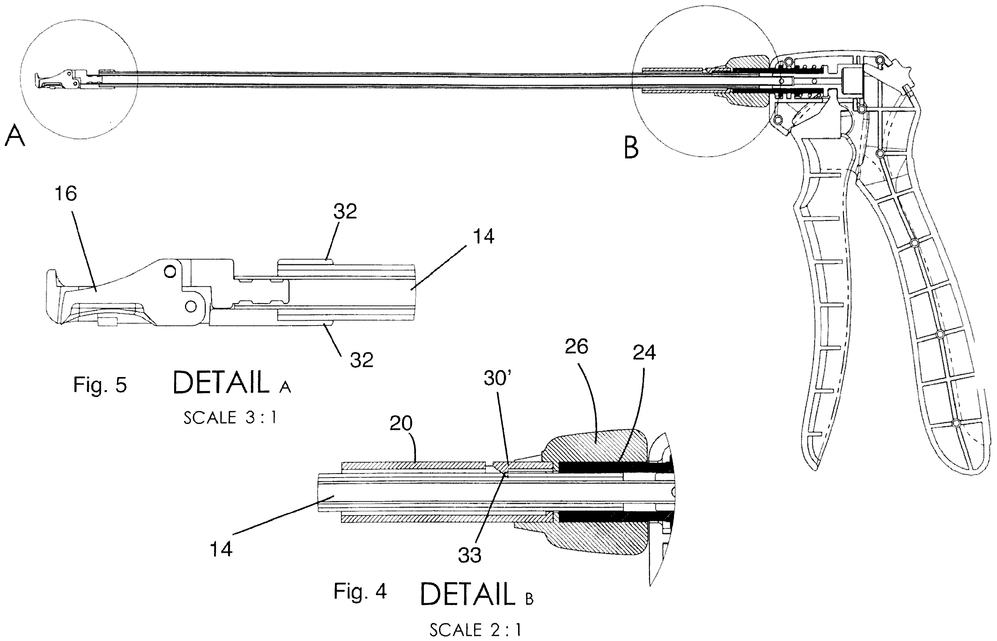

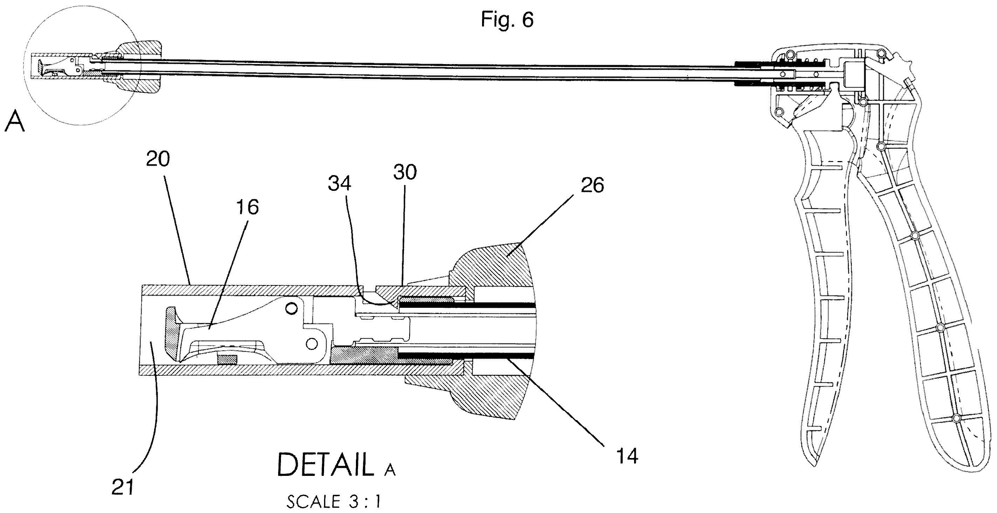

Figure 4 illustrates a cross-section through the safety sleeve 20 in the retracted position. Internally, the safety sleeve 20 comprises a substantially cylindrical channel 21 shaped to accommodate the elongate shaft 14, along with plurality of resilient clips 30 which are biased towards the shaft 14 when the safety sleeve 20 is in the retracted position. As shown in Figure 5, the distal end of the elongate shaft 14 includes a collar 32 which has increased diameter compared to the remainder of the shaft 14. The collar 32 is located adjacent the cutting tip. The clip or clips 30 have an enlarged end in the form of a lip 33. After the apparatus 10 has been used to remove a biopsy sample from a patient, the health professional slides the safety sleeve 20 along the elongate shaft 14 towards the cutting tip 16. The resilient clips 30 ride over the collar 32, coming to rest on the distal side of the collar 32. As shown in Figure 6, the lip 33 of the clip then rests on the distal side of the collar 32, preventing retraction of the safety sleeve back along the elongate shaft. The collar 32 also prevents complete removal of the safety sleeve 20 from the elongate shaft, since the base of the sleeve butts up against the other side of the ridge. By locking the safety sleeve 20 in position over the cutting tip, the health professional is protected from any accidental contact with the cutting tip.

Any means allowing cooperation between the distal end of the elongate shaft and the safety sleeve which enables the sleeve to be locked into position over the cutting tip fulfills the object of the invention. This could include a combination of resilient clips on the sleeve and an aperture on the shaft into which the clips, or protrusions on the clips locate.

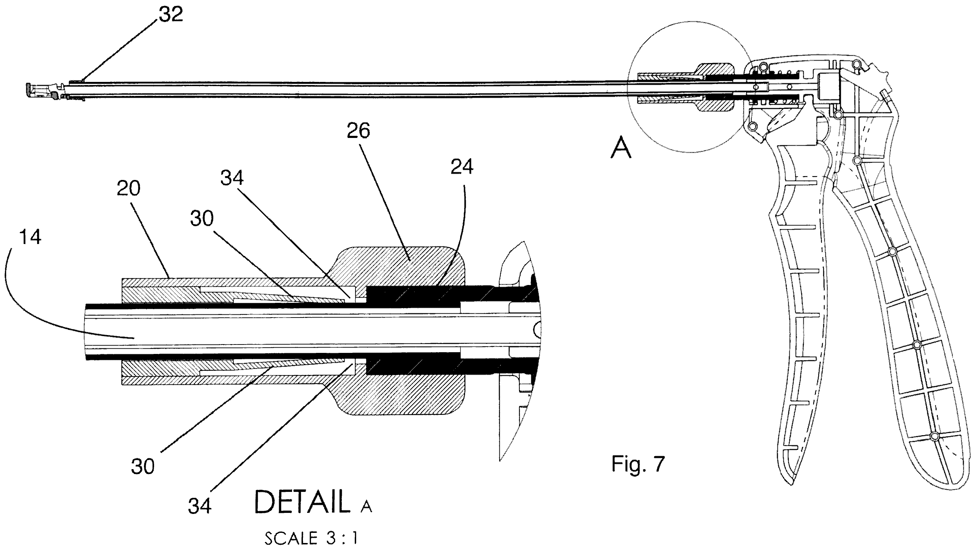

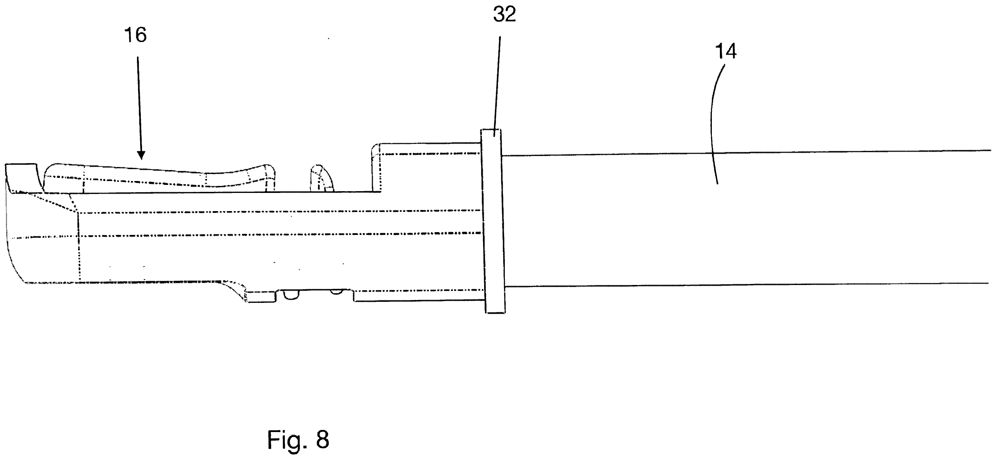

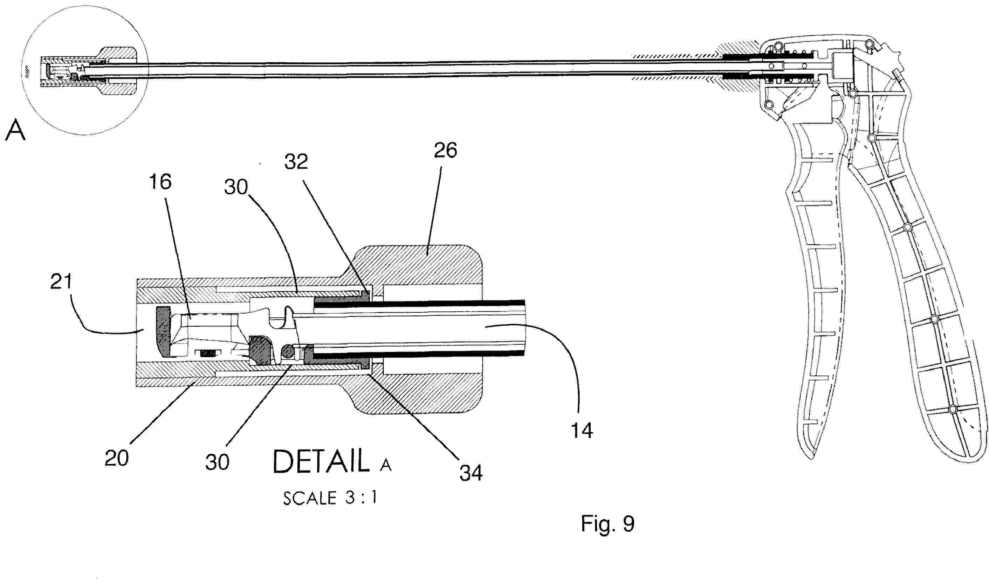

Figure 7 illustrates a cross-section through an alternative embodiment of the invention with the safety sleeve 20 in the retracted position. As with the previous example, internally, the safety sleeve 20 comprises a substantially cylindrical channel 21 shaped to accommodate the elongate shaft 14, however in this example the locking mechanism comprises a plurality of resilient tabs 31 which are biased towards the shaft 14 when the safety sleeve 20 is in the retracted position. As shown in Figure 8, the distal end of the elongate shaft 14 includes a narrow collar 32' which has increased diameter compared to the remainder of the shaft 14. The collar 32' is located adjacent the cutting tip. Internally, as shown in Figure 7, the safety sleeve 20 also comprises an annular groove or channel 34 shaped to receive the collar 32' of the shaft 14. After the apparatus 10 has been used to remove a biopsy sample from a patient, the health professional slides the safety sleeve 20 along the elongate shaft 14 towards the cutting tip 16. The resilient tabs 31 ride over the collar 32', coming to rest on the distal side of the ridge 32'. The collar 32' then comes to rest in the channel 34, as shown in Figure 9 and the safety sleeve 20 is now prevented from axial movement along the shaft 14 in either direction. By locking the safety sleeve 20 in position over the cutting tip, the user is protected from any accidental contact with the cutting tip and subsequent injury.

The lockable safety sleeve arrangement could be used in conjunction with any surgical cutting instrument which includes a cutting element at the end of an elongate shaft.

## Claims

1. A surgical cutting instrument comprising a handle portion (12) and an elongate shaft (14), the shaft having a distal end and a proximal end and a longitudinal axis extending therebetween, the shaft mounting a cutting element (16) at the distal end, the instrument further comprising a safety sleeve (20)
including a substantially cylindrical channel through which the elongate shaft extends, wherein the safety sleeve is moveable axially along the shaft between a retracted position in which the cutting tip is exposed, and an extended position in which the cutting tip is located within the cylindrical channel of the safety sleeve;
wherein the instrument further comprises locking means (30,32) for preventing further axial movement of the safety sleeve with respect to the elongate shaft when the safety sleeve is in the extended position; wherein the locking means automatically engages when the safety sleeve is in the extended position, and the locking means prevents movement of the safety sleeve from the extended position to the retracted position; and wherein the locking means comprises first and second cooperating elements (30,32) each located on a respective one of the distal end of the shaft and the safety sleeve.

2. A surgical cutting instrument according to Claim 1, wherein the shaft is rotatably mounted onto the handle portion at the proximal end, allowing for rotation of the shaft about the longitudinal axis.

3. A surgical cutting instrument according to Claim 2, wherein, when the safety sleeve is in the retracted position, the safety sleeve is configured to rotate the elongate shaft.

4. A surgical cutting instrument according to Claim 2 or 3, wherein the instrument further comprises means for maintaining a static connection between the safety sleeve and the elongate shaft, and wherein the means for maintaining a static connection comprises third and fourth cooperating elements (22,24) each located on a respective one of the proximal end of the shaft and the safety sleeve.

5. A surgical cutting instrument according to Claim 4, wherein rotation of the safety sleeve effects rotation of the elongate shaft when the safety sleeve is in the retracted position

6. A surgical cutting instrument according to Claim 4 or 5, wherein the third and fourth cooperating elements comprise male/female cooperating elements.

7. A surgical cutting instrument according to Claim 6, wherein one of the third and fourth cooperating elements is a protrusion (24) and the other of the third and fourth cooperating elements is a slot (22) shaped to accommodate the protrusion.

8. A surgical cutting instrument according to any preceding Claim, wherein the first cooperating means comprises: a collar (32) located at the distal end of the elongate shaft, adjacent the cutting tip; at least one resilient tab (30) located on the safety sleeve; and an annular channel shaped to receive the collar located within the cylindrical channel of the safety sleeve.

9. A surgical cutting instrument according to Claim 8, wherein the at least one resilient tab includes a protrusion (33) and one edge of the annular channel is defined by the protrusion.

10. A surgical cutting instrument according to any of Claims 1 to 7, wherein the first cooperating element comprises at least one resilient clip (30) including a protrusion (33) located on the safety sleeve and biased towards the elongate shaft; and the second cooperating element comprises at least one aperture for receiving the or each protrusion located at the distal end of the elongate shaft, adjacent the cutting tip, such that in the extended position the or each protrusion locates within the or each aperture preventing further axial movement of the safety sleeve with respect to the elongate shaft.

11. A surgical cutting instrument according to any preceding Claim, wherein the safety sleeve further comprises a portion of increased diameter (26) located adjacent the proximal end of the elongate shaft.

12. A surgical cutting instrument according to Claim 11, wherein the portion of the safety sleeve with increased diameter is configured to be gripped and includes a plurality of ridges (28).

13. A surgical cutting instrument according to any preceding claim for extracting a biopsy sample.

## Patentansprüche

1. Chirurgisches Schneidinstrument, umfassend einen Griffteil (12) und einen länglichen Schaft (14), wobei der Schaft ein distales Ende und ein proximales Ende und eine Längsachse, die sich dazwischen erstreckt, aufweist, wobei der Schaft ein Schneidelement (16) an dem distalen Ende montiert, wobei das Instrument weiterhin eine Sicherheitshülse (20) umfasst, das einen im Wesentlichen zylindrischen Kanal beinhaltet, durch den sich der längliche Schaft erstreckt, wobei die Sicherheitshülse axial entlang des Schafts zwischen einer eingefahrenen Position, in der die Schneidspitze freigelegt ist, und einer ausgefahrenen Position, in der die Schneidspitze sich innerhalb des zylindrischen Kanals der Sicherheitshülse befindet, bewegbar ist;
wobei das Instrument weiterhin ein Arretiermittel (30, 32) zur Verhinderung einer weiteren axialen Bewegung der Sicherheitshülse in Bezug auf den länglichen Schaft, wenn die Sicherheitshülse in der ausgefahrenen Position ist, umfasst; wobei das Arretiermittel sich automatisch einrastet, wenn die Sicherheitshülse in der ausgefahrenen Position ist, und das Arretiermittel eine Bewegung der Sicherheitshülse aus der ausgefahrenen Position in die eingefahrene Position verhindert; und wobei das Arretiermittel ein erstes und ein zweites zusammenwirkendes Element (30, 32) umfasst, die sich jeweils auf einem bzw. einer jeweiligen von dem distalen Ende des Schafts und der Sicherheitshülse befinden.

2. Chirurgisches Schneidinstrument nach Anspruch 1, wobei der Schaft drehbar auf dem Griffteil an dem proximalen Ende montiert ist, was eine Drehung des Schafts um die Längsachse ermöglicht.

3. Chirurgisches Schneidinstrument nach Anspruch 2, wobei, wenn die Sicherheitshülse in der eingefahrenen Position ist, die Sicherheitshülse dazu konfiguriert ist, den länglichen Schaft zu drehen.

4. Chirurgisches Schneidinstrument nach Anspruch 2 oder 3, wobei das Instrument weiterhin ein Mittel zum Aufrechterhalten einer statischen Verbindung zwischen der Sicherheitshülse und dem länglichen Schaft umfasst, und wobei das Mittel zum Aufrechterhalten einer statischen Verbindung ein drittes und ein viertes zusammenwirkendes Element (22, 24) umfasst, die sich jeweils auf einem bzw. einer jeweiligen von dem proximalen Ende des Schafts und der Sicherheitshülse befinden.

5. Chirurgisches Schneidinstrument nach Anspruch 4, wobei eine Drehung der Sicherheitshülse eine Drehung des länglichen Schafts bewirkt, wenn die Sicherheitshülse in der eingefahrenen Position ist.

6. Chirurgisches Schneidinstrument nach Anspruch 4 oder 5, wobei das dritte und das vierte zusammenwirkende Element ein männliches/ein weibliches zusammenwirkendes Element umfassen.

7. Chirurgisches Schneidinstrument nach Anspruch 6, wobei eines von dem dritten und dem vierten zusammenwirkenden Element ein Vorsprung (24) ist und das andere von dem dritten und dem vierten zusammenwirkenden Element ein Schlitz (22) ist, der geformt ist, um den Vorsprung aufzunehmen.

8. Chirurgisches Schneidinstrument nach einem vorhergehenden Anspruch, wobei das erste zusammenwirkende Mittel umfasst: einen Kragen (32), der sich an dem distalen Ende des länglichen Schafts befindet, benachbart zu der Schneidspitze; mindestens eine federnde Lasche (30), die sich auf der Sicherheitshülse befindet; und einen ringförmigen Kanal, der geformt ist, um den Kragen aufzunehmen, der sich innerhalb des zylindrischen Kanals der Sicherheitshülse befindet.

9. Chirurgisches Schneidinstrument nach Anspruch 8, wobei die mindestens eine federnde Lasche einen Vorsprung (33) beinhaltet und eine Kante des ringförmigen Kanals von dem Vorsprung definiert wird.

10. Chirurgisches Schneidinstrument nach einem der Ansprüche 1 bis 7, wobei das erste zusammenwirkende Element mindestens eine federnde Klammer (30) umfasst, die einen Vorsprung (33) beinhaltet, der sich auf der Sicherheitshülse befindet und zu dem länglichen Schaft hin vorgespannt ist, und das zweite zusammenwirkende Element mindestens eine Öffnung zum Aufnehmen des oder jedes Vorsprungs umfasst, der sich an dem distalen Ende des länglichen Schafts befindet, benachbart zu der Schneidspitze, so dass der oder jeder Vorsprung sich in der ausgefahrenen Position innerhalb der oder jeder Öffnung anordnet, wodurch eine weitere axiale Bewegung der Sicherheitshülse in Bezug auf den länglichen Schaft verhindert wird.

11. Chirurgisches Schneidinstrument nach einem vorhergehenden Anspruch, wobei die Sicherheitshülse weiterhin einen Abschnitt mit einem erhöhten Durchmesser (26) umfasst, der benachbart zu dem proximalen Ende des länglichen Schafts befindet.

12. Chirurgisches Schneidinstrument nach Anspruch 11, wobei der Abschnitt der Sicherheitshülse mit einem erhöhten Durchmesser dazu konfiguriert ist, gegriffen zu werden, und eine Vielzahl von Rippen (28) beinhaltet.

13. Chirurgisches Schneidinstrument nach einem vorhergehenden Anspruch zum Extrahieren einer Biopsieprobe.

## Revendications

1. Un instrument de coupe chirurgical comprenant une partie manche (12) et une tige allongée (14), la tige ayant une extrémité distale et une extrémité proximale et un axe longitudinal s'étendant entre celles-ci, la tige ayant un élément de coupe (16) monté à l'extrémité distale, l'instrument comprenant en outre un manchon de sécurité (20) incluant un canal sensiblement cylindrique à travers lequel s'étend la tige allongée, dans lequel le manchon de sécurité est mobile axialement le long de la tige entre une position rétractée dans laquelle la pointe de coupe est exposée, et une position étendue dans laquelle la pointe de coupe est située à l'intérieur du canal cylindrique du manchon de sécurité ;
dans lequel l'instrument comprend en outre un moyen de verrouillage (30, 32) pour interdire tout autre déplacement axial du manchon de sécurité par rapport à la tige allongée quand le manchon de sécurité est à la position étendue ;
dans lequel le moyen de verrouillage se met en prise automatiquement quand le manchon de sécurité est à la position étendue, et le moyen de verrouillage interdit le déplacement du manchon de sécurité de la position étendue à la position rétractée ; et
dans lequel le moyen de verrouillage comprend des premier et deuxième éléments coopérants (30,32) situés chacun respectivement sur l'extrémité distale de la tige et sur le manchon de sécurité.

2. Un instrument de coupe chirurgical selon la revendication 1, dans lequel la tige est montée rotative sur la partie manche à l'extrémité proximale, ce qui permet la rotation de la tige autour de l'axe longitudinal.

3. Un instrument de coupe chirurgical selon la revendication 2, dans lequel, quand le manchon de sécurité est à la position rétractée, le manchon de sécurité est configuré pour faire tourner la tige allongée.

4. Un instrument de coupe chirurgical selon la revendication 2 ou 3, dans lequel l'instrument comprend en outre un moyen pour maintenir une liaison statique entre le manchon de sécurité et la tige allongée, et dans lequel le moyen de maintien d'une liaison statique comprend des troisième et quatrième éléments coopérants (22, 24) situés chacun respectivement sur l'extrémité proximale de la tige et sur le manchon de sécurité.

5. Un instrument de coupe chirurgical selon la revendication 4, dans lequel la rotation du manchon de sécurité affecte la rotation de la tige allongée quand le manchon de sécurité est à la position rétractée.

6. Un instrument de coupe chirurgical selon la revendication 4 ou 5, dans lequel les troisième et quatrième éléments coopérants comprennent des éléments coopérants mâle / femelle.

7. Un instrument de coupe chirurgical selon la revendication 6, dans lequel un des troisième et quatrième éléments coopérants est une saillie (24) et l'autre des troisième et quatrième éléments coopérants est une fente (22) ayant une forme pour accueillir la saillie.

8. Un instrument de coupe chirurgical selon l'une quelconque des revendications précédentes, dans lequel le premier moyen coopérant comprend : un collet (32) situé à l'extrémité distale de la tige allongée, adjacent à la pointe de coupe ; une ou plusieurs languettes élastiques (30) situées sur le manchon de sécurité ; et un canal annulaire ayant une forme pour recevoir le collet situé à l'intérieur du canal cylindrique du manchon de sécurité.

9. Un instrument de coupe chirurgical selon la revendication 8, dans lequel la ou les languettes élastiques incluent une saillie (33) et un des bords du canal annulaire est défini par la saillie.

10. Un instrument de coupe chirurgical selon l'une quelconque des revendications 1 à 7, dans lequel le premier élément coopérant comprend une ou plusieurs pinces élastiques (30) incluant une saillie (33) situées sur le manchon de sécurité et sollicitées vers la tige allongée ; et le deuxième élément coopérant comprend une ou plusieurs ouvertures pour recevoir la ou les saillies situées à l'extrémité distale de la tige allongée, adjacentes à la pointe de coupe, de telle sorte que, à la position étendue, la ou les saillies se situent à l'intérieur de la ou des ouvertures interdisant tout autre déplacement axial du manchon de sécurité par rapport à la tige allongée.

11. Un instrument de coupe chirurgical selon l'une quelconque des revendications précédentes, dans lequel le manchon de sécurité comprend en outre une partie augmentée en diamètre (26) située adjacente à l'extrémité proximale de la tige allongée.

12. Un instrument de coupe chirurgical selon la revendication 11, dans lequel la partie du manchon de sécurité étant augmentée en diamètre est configurée pour être saisie et inclut une pluralité de nervures (28).

13. Un instrument de coupe chirurgical selon l'une quelconque des revendications précédentes destiné à prélever un échantillon de biopsie.
